Europäisches Patentamt

European Patent Office

Office européen des brevets

(11) Publication number: **0 371 173**
A1

# EUROPEAN PATENT APPLICATION

(21) Application number: **88310339.2**

(22) Date of filing: **03.11.88**

(51) Int. Cl.⁵: **A61M 1/36, A61M 1/16,
A61M 1/32**

(43) Date of publication of application:
**06.06.90 Bulletin 90/23**

(84) Designated Contracting States:
**DE FR GB**

(71) Applicant: **BAXTER INTERNATIONAL INC. (a
Delaware corporation)
One Baxter Parkway
Deerfield Illinois 60015(US)**

(72) Inventor: **Bringham, Richard Larsen
1202 Pavoreal
San Clemente, CA 92672(US)**

(74) Representative: **MacGregor, Gordon et al
ERIC POTTER & CLARKSON St. Mary's Court
St. Mary's Gate
Nottingham, NG1 1LE(GB)**

(54) **Integral blood defoamer, reservoir, heat exchanger, and cardiotomy filter.**

(57) A device for heating, defoaming, temporarily storing, and oxygenating venous blood incorporates a cardiotomy stream filter for filtering a cardiotomy stream and directing the cardiotomy stream onto the surface of the heating coil prior to fully mixing with the venous stream to avoid temperature fluctuation in the venous or arterial blood flow. The cardiotomy filter includes a vertical perforated inlet tube surrounded by a tubular defoamer and a tubular pleated filter all sealed and supported at upper and lower ends by a retainer, and is nested within a heating coil of the device. A bottom disc directs the output of the cardiotomy filter onto the heating coil.

FIG. 1

EP 0 371 173 A1

# INTEGRAL BLOOD DEFOAMER, RESERVOIR, HEAT EXCHANGER, AND CARDIOTOMY FILTER

## Technical Field

The invention relates to apparatus for filtering, temperature control, defoaming and storing blood in an extracorporeal circuit, and particularly to an integral apparatus combining processing and handling of a cardiotomy stream with a venous stream.

## Description of the Prior Art

In surgical procedures such as open-heart surgery, an extracorporeal circuit including a pump and an oxygenator is used to replace the functions of the lungs and heart for oxygenating the blood flow from the veins and for pumping the oxygenated blood into the arteries. The extracorporeal circuit generally includes provision for reservoiring the blood flow to accommodate fluctuations in venous blood flow, for defoaming the blood flow to remove gas bubbles which evolve or are induced in the extracorporeal circuit, and for controlling the temperature of the blood to account for heat losses in the extracorporeal circuit or to temporarily reduce and then raise body temperature. Additionally, the surgical procedures result in bleeding which is suctioned from the incision site to form a cardiotomy bloodstream. This cardiotomy stream is generally filtered to remove particulate material suctioned from the incision site, reservoired, and combined with the venous or arterial flow in the extracorporeal circuit.

The prior art, as exemplified in U.S. Patents 4,205,042 and 4,490,331, contains integral apparatus incorporating a cardiotomy filter for filtering a cardiotomy stream along with a reservoir, a defoamer, blood temperature control facilities, and a bubbler-type or membrane-type oxygenator. The cardiotomy stream, after filtering, is combined with either the venous blood (prior to oxygenation) or the arterial blood (after oxygenation). The blood temperature control facilities include a heat exchanger for either the venous flow or the combined flow.

Generally, the prior art blood oxygenators with integral cardiotomy filters have one or more deficiencies, such as producing restriction to blood flow requiring excessive blood pressures and cell injury, requiring separate storage or reservoir space for the cardiotomy stream, having a structure which is difficult and expensive to manufacture, etc.

## Summary of the Invention

The invention is summarized in a reservoir, heating, defoaming and cardiotomy filtering device for an extracorporeal blood system, including a bowl-like housing forming a blood reservoir and having a bottom with a blood outlet and a top with a venous blood inlet and a central cardiotomy blood inlet, a heat exchanger coil mounted within the housing and having a plurality of convolutions formed around a vertical axis, facilities for directing blood flow from the venous inlet in an annular pattern over the upper convolution of the heat exchanger coil, a defoamer mounted within the housing and interposed between the heat exchanger and the blood outlet to remove gas bubbles in the blood passing to the blood outlet, a cardiotomy filter connected to the cardiotomy inlet and mounted within the housing so as to extend downward from the top into the convolutions of the heat exchanger coil for receiving and filtering a cardiotomy stream, and facilities for directing the filtered cardiotomy bloodstream onto the convolutions of the heat exchanger coil.

An object of the invention is to construct a new and improved device which combines the filtering and temporary storage of a cardiotomy stream with the temporary storage, heating and defoaming of the venous stream.

Another object of the invention is to provide for heating of the cardiotomy stream in an integrated reservoir, heating, defoaming and cardiotomy filtering device.

One advantage of the invention is that a blood oxygenator with temporary storage and defoaming can include a cardiotomy filter with relatively little modification and additional expense.

Another advantage of the invention is that heating of the cardiotomy stream reduces trauma on blood cells that occur when a cool non-heated cardiotomy stream is introduced to a venous or arterial stream.

Other objects, advantages and features of the invention will be apparent from the following description taken in conjunction with the accompanying drawings.

## Brief Description of the Drawings

Fig. 1 is a vertical cross-section of a blood oxygenator including a reservoir, heating, defoaming and cardiotomy filtering device in accordance with the invention.

Fig. 2 is a top view of the oxygenator of Fig. 1.

Fig. 3 is a top view of a cardiotomy filter holder in the oxygenator of Fig. 1.

Fig. 4 is a bottom view of the oxygenator of Fig. 1.

Fig. 5 is an elevational view of the holder of Fig. 3.

Fig. 6 is an isometric view of a pleated filter material for being assembled into a tubular filter for insertion in the holder of Figs. 3 and 5 to form a cardiotomy filter structure.

Fig. 7 is an enlarged cross-sectional view of a broken away portion of the filter material of Fig. 6.

Fig. 8 is a top view of the holder of Fig. 3 with the pleated filter of Fig. 6 installed therein.

Fig. 9 is a top view of the filter of Fig. 6 shown in an assembled tubular form for being inserted into the holder of Figs. 3 and 5.

Fig. 10 is a vertical section view of a modified blood oxygenator similar to that of Fig. 1, but modified for pediatric use.

Fig. 11 is an elevational section view of a bubbler-type variation of a blood oxygenator with a reservoir, heating, defoaming, blood oxygenating and cardiotomy filtering device in accordance with the invention.

Fig 12 is a top view of the oxygenator of Fig. 11.

Description of the Preferred Embodiments

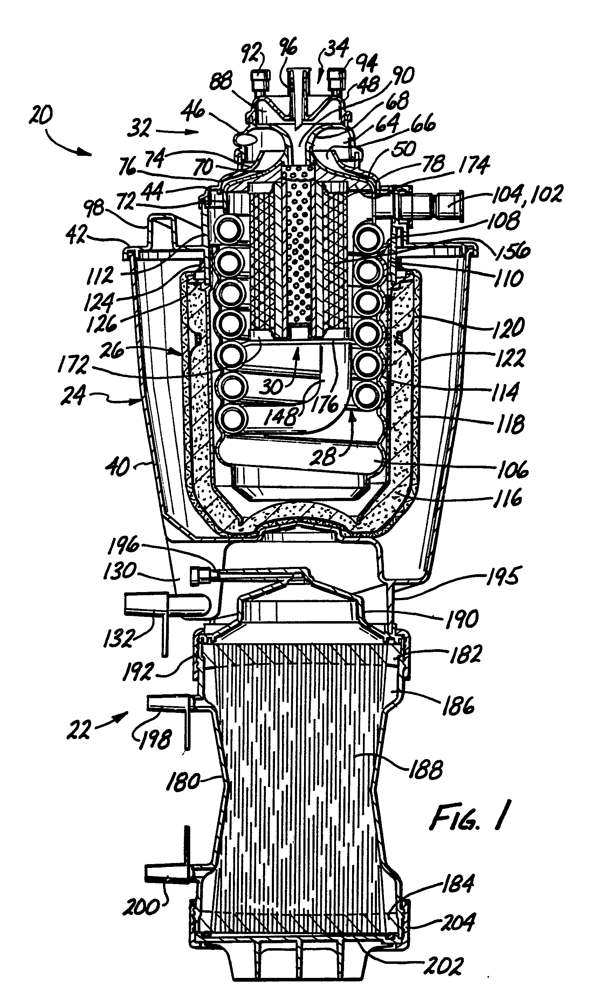

As shown in Fig. 1, one embodiment in accordance with the invention includes a reservoir, defoamer, heat exchanger and cardiotomy filter device indicated generally at 20 mounted on top of a membrane-type filter indicated generally at 22. The upper device 20 is formed from a nested arrangement of an outer hard shell housing 24 forming a reservoir, a defoamer indicated generally at 26, a heat exchanger coil indicated generally at 28 having vertically arranged coils, and a cardiotomy filter indicated generally at 30. An annular venous blood inlet facility indicated generally at 32 is formed in the top of the unit 20 for directing incoming blood flow to the upper convolution 32 of the heat exchanger coil 28. A central cardiotomy blood inlet facility indicated generally at 34 is formed on the top of the unit for directing a cardiotomy blood stream into the filter 30.

The reservoir 24 includes a molded thermoplastic bowl-shaped container portion 40 and a cover formed by molded thermoplastic parts 42, 44, 46, 48 and 50. The parts 42, 44, 46, 48 and 50 are secured together by conventional means such as being induction thermally bonded together utilizing a commercial iron containing bonding agent, solvent bonding, adhesive bonding, RF bonding, ultrasonic bonding, vibration bonding, or the like. The parts 42, 44, 46 and 48 have mating annular tongue and groove arrangements formed thereon to enhance the strength and sealing of the bonds. The part 42 is shown mounted on the housing 40 using a rotating seal of the type disclosed in U.S. Patent No. 4,440,723 so that the relative orientation of the upper assembly can be adjusted relative to the housing 40; however, a fixed joint such as that used between parts 42, 44, 46 and 48 may be used instead of the rotating seal.

As shown in Fig. 2, the venous blood inlet facility 32 is formed by part 46 which has tangential inlet connectors 54 and 56 (illustrated with sealing caps positioned thereon). Sampling ports 58 and 60 are provided on the connectors 54 and 56, and a temperature probe 62 is provided on the connector 54. The inlets 54 and 56 open tangentially into an inlet chamber 64, Fig. 1, which communicates with a lower annular opening 66 defined by parts 44 and 50. The part 50 is mounted on a center tubular projection 68 extending downward from the top of part 46, and has a downward and outwardly flaring skirt portion 70 which at the upper end defines the inner edge of the annular opening 66. As more particularly disclosed in U.S. patent application No. 565,236, filed December 27, 1983, the skirt portion 70 terminates in a downward vertical cylindrical portion 72 which extends to the top center of the surface of the tube forming the upper convolution of the coil 28 for directing flow of blood thereto. The part 44 has an inner upward flaring flange 74 defining the outer edge of the annular opening 66, and extends outwardly and downwardly to a vertical cylindrical portion 76 which cooperates with the vertical cylindrical portion 72 of the member 50 for forming a narrow annular opening 78, approximately 0.050 inch (1.3 mm) wide to provide a film-like flow which will follow the downward extending cylindrical portion 72 to the top convolution of the coil 28.

The cardiotomy blood inlet facility 34 is formed by upper part 48 which has inlets 82, 84 and 86, Fig. 2, opening into a chamber 88 formed between members 46 and 48. The chamber 88 communicates at its bottom with the passage 90 through the tubular projection 68 which delivers the blood flow from chamber 88 into the filter 30. Ports 92 and 94 are provided for the inlets 82 and 86 and a port 96 is provided for the center of the chamber 88.

The cover part 42 is provided with a raised portion 98 in which is mounted a port or vent 100 for providing an exhaust from the reservoir to remove gas which is carried or evolves from the blood flow input.

The heat exchanger coil 28 is a coiled, bellowed, aluminum heat exchanger tube which has respective inlet and outlet openings 102 and 104 secured by appropriate mating openings in the parts 42, 44 and 50. The outer surface of the aluminum coil 28 is preferably annodized and/or coated with a commercial urethane-based biocoat, epoxy or other thin coating of biocompatible material applied by a suitable technique such as electrostatic powder coating. Alternatively, the coil 28 may be stainless steel, and the coil may be formed from a tube which is smooth, fluted or formed with an alternative extended surface.

A rigid housing 106 has a helical scallop mating with the exterior of the coil 28 for maintaining the blood flow over the heat exchanger coils 28. The housing 106 has an outward extending lip 108 which engages projections 110 formed on an inner wall 112 of the top part 42, and is secured and sealed thereto by a suitable adhesive.

The defoamer 26, which is described in more detail in U.S. patent application Serial No. 06/885,963, filed on July 14, 1986, has a center tubular grid-like support 114 mounted on the bottom edge of the inner wall 112 of member 42. An open cell polyurethane sock 116 is mounted over the grid support 114 which in turn has a woven polyester screen sock secured on the lower three-fourths portion thereof. An outer sock 120 of open cell polyurethane, with larger pores than the sock 116, surrounds the inner socks 116 and 118 and is secured by a knit polyester sock 122 which is fastened at its upper end by a tie 124 above an outwardly extending lip 126 of the support 114 for securing the sock on the grid 114.

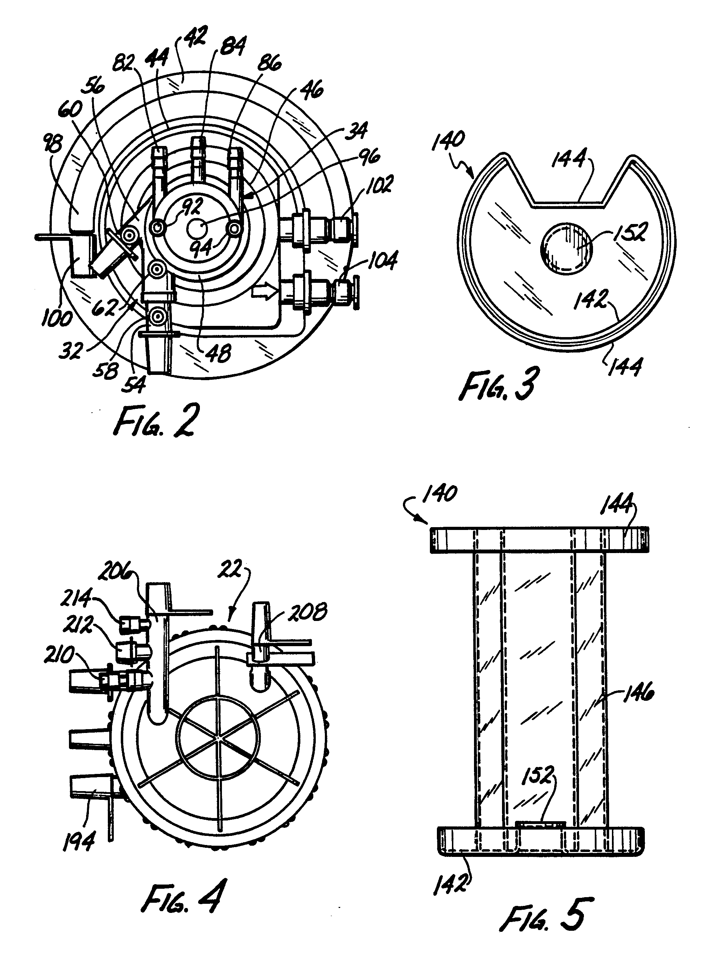

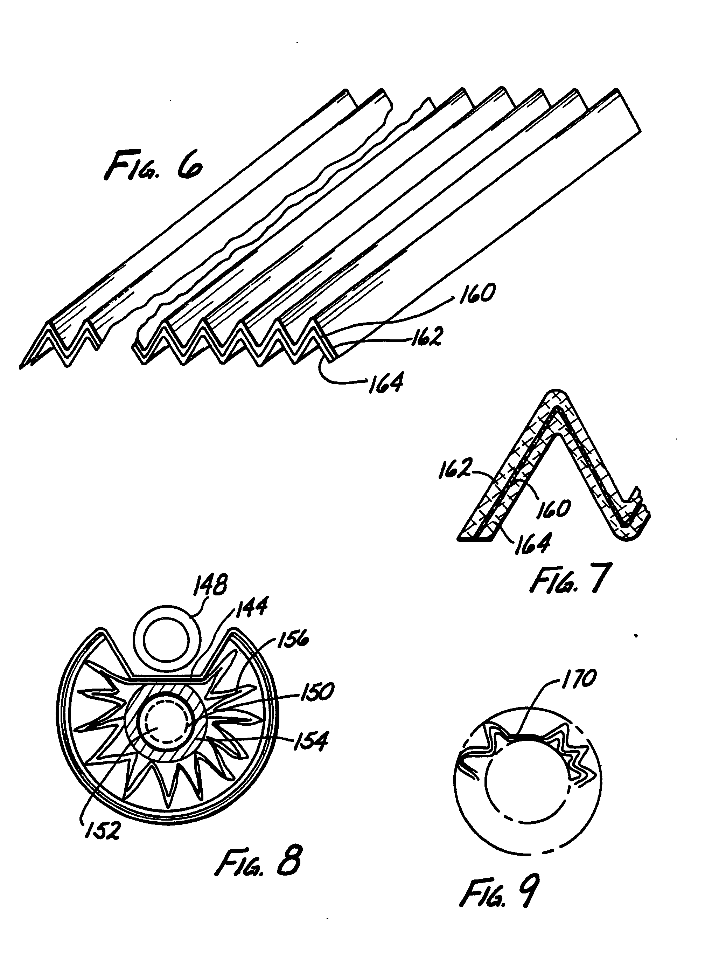

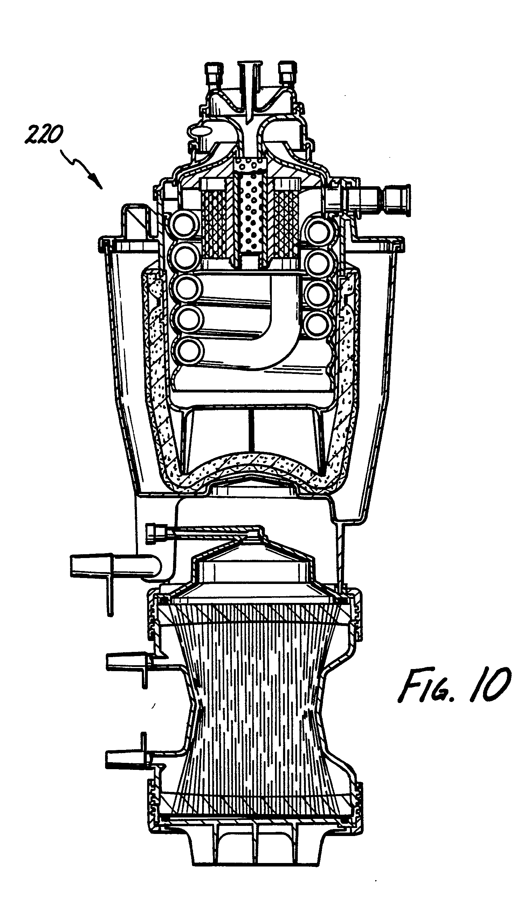

The filter 30 has a support indicated generally at 140 in Figs. 3 and 5 which includes a bottom tray portion 142 and an upper ring portion 144 joined by a vertical panel portion 146 which is adapted to accommodate a vertical section 148, Fig. 1, of the heat exchanger tube extending vertically in the coil 28 to connect the bottom convolution of the coil to the heating fluid outlet 104. As shown in Figs. 1 and 8, the filter has a perforated inner vertical tube 150 which is secured at its lower end on a raised portion 152 of the tray 144 and receives the tubular projection 68 at its upper end. A tubular defoamer layer, such as open cell polyurethane foam 154 surrounds the tube 150. A pleated filter 156, retained by the upper ring 144 and the lower tray 142 surrounds the defoamer member 154. The filter 156 is formed from a pleated material which, as shown in Figs. 6 and 7, has a center filter layer 160 sandwiched between two support layers 162 and 164. The filter material 160 is preferably a woven polyester material of about 20 micron mesh, while the outer layers 162 and 164 are suitable support material, such as an extruded netting for retaining the inner filter layer 160. Sheets of the materials 160, 162 and 164 are assembled and pleated as shown in Fig. 6, care being taken to avoid fracturing the materials of the layers 160, 162 and 164. The pleated structure generally holds the layers together. The pleated sheet of Fig. 6 is then folded into a tubular shape, as shown in Fig. 9 and overlapping end portions 170 are bonded together by heat sealing, hot melt adhesive, or other securing means to secure the pleated sheet into the tubular configuration. This pleated tubular configuration of Fig. 9 is then inserted into the holder 140 of Figs. 3 and 5 over the defoamer 154. As shown in Fig. 1, the lower end of the filter 156 is sealed by potting material 172, and the upper end of the filter 156 as well as the upper ring 144 is secured and sealed in the member 50 by potting material 174. The potting materials 172 and 174 are preferably a hot melt bonding material, or other suitable potting material.

A flexible disc, such as foam polyurethane disc 176 (Fig. 1), is secured, such as by hot melt adhesive or other securing means, to the bottom of the support tray 142. The disc 176 has a diameter extending from the filter 30 into engagement with the coil 28 so as to direct the flow of the cardiotomy stream from the filter 30 onto the convolutions of the coil 28.

The membrane oxygenator 22 is described in detail in U.S. patent application Serial No. 06/885,207, filed July 14, 1986, and includes a molded vertical tubular housing 180 with upper and lower seals 182 and 184 forming a chamber 186 through which a multiplicity of hollow membrane fibers 188 extend and have upper ends secured in the seal 122 and lower ends secured in the seal 184. An upper cap 190 is secured to the top side of the seal 184 by a threaded retaining ring 192 and has a tangential inlet 194, see Fig. 4, for receiving blood which then is distributed to the open upper ends of the hollow membrane fibers 188. The upper cap 190 also has a groove for receiving and being secured such as by an adhesive to a mounting flange 195 of the upper device 20 to mount the membrane oxygenator unit 22 on the bottom of the upper device 20. A vent 196 is provided in the top of the cap 190 for removing gas from the cap 190. Inlet 198 and outlet 200 communicate with the chamber 186 for passing air, oxygen or an oxygen-rich gas through the chamber 186 so as to oxygenate the blood flowing through the membrane fibers 188 and to remove carbon dioxide therefrom. A lower cap 202 is securely fastened and sealed to the outer periphery of the lower seal 184 by a threaded retainer ring 204 for receiving the blood flow and discharging the blood flow through one or more of the outlets 206 and 208, see Fig. 4. A temperature probe port 210 and sample or branch outlet ports 212 and 214 are provided on the main outlet 206.

The various molded parts such as the reservoir shell 40, the top parts 42, 44, 46, 48 and 50, housing 106, support 140, perforated tube 150, the tubular housing 180, upper and lower caps 190 and 202 and the retaining rings 192 and 204 are made from a clear polymer material such as polycarbonate, acrylic, ABS, SAN or other suitable material. Where appropriate, various parts, such as the receptacle shell 40, cover parts 42, 44, 46, 48 and 50, housing 106, support 140, tube 150, caps 190 and 202, membrane fibers 188, seals 182 and 184, and other parts, may be coated with a conventional anti-thrombogenic material to avoid forming clots in the blood being processed. Preferably, the defoamer sleeve 154 and the upper one-third portion of the defoamer sock 116 are coated with a conventional silicone antifoam compound.

In use of the oxygenator of Fig. 1, the upper and lower units will be initially primed by passing a priming fluid, such as the patient's blood or other compatible fluid, into one or more of the venous inlets 54 and 56 and one or more of the cardiotomy inlets 82, 84 and 86. The priming fluid after cascading down the heating coils 28 passes through the defoamer 26 and into the bottom of the reservoir 40 where the priming fluid is removed through the connector 132 through a pump (not shown) which then returns the fluid to the inlet 194, Fig. 4, of the membrane oxygenator 22. The priming fluid passes into the inlet cap 190 and flushes gas from the membrane fibers 188 and the outlet cap 202, as well as the outlets 206 and 208. The system is then connected for operation normally where venous blood flow flows through inlet 54, Fig. 2, into the chamber 64, Fig. 1, and is then distributed over the upper convolution of coil 28 and cascades down over each lower convolution to obtain desired blood temperature. Water is heated or cooled and passed through inlet 102 and out outlet 104.

The cardiotomy stream from one or more suction pumps (not shown), enters one or more of the inlets 82, 84 and 86 and passes through the passageway 90 into the center tube 150 of the filter 30. The cardiotomy stream passes through the apertures in the tube 150 and then is defoamed by contact in passing through the defoamer material 154. Thereafter the blood passes through the pleated filter 156 to remove particles and other materials which have a size greater than about 20 microns. After passing through the filter material 156, the blood is directed by the disc 176 onto the coil convolutions 28. This insures that the cardiotomy stream is also maintained at the desired temperature along with the venous stream. The cardiotomy stream, being removed from the incision site oan be substantially cooler than the venous stream. Insuring that the cardiotomy stream contacts the heating coil directly prior to being fully mixed with the venous stream insures that the cardiotomy stream is raised to the desired temperature so as to avoid a shock by a sudden temperature change in the fluid surrounding blood cells in the venous or arterial stream which can occur when a non-heated cardiotomy stream is added to the venous or arterial stream.

The nested arrangement of the cardiotomy filter 30 within the coil 28 which is in turn nested within the defoamer 26 nested within the reservoir 40 results in a relatively compact unit which avoids large elevational changes in the blood being passed through the extracorporeal circuit. This helps maintain the pressure in the blood flow at a minimum to avoid damage to the blood that may be caused by such pressure.

The particular stucture of the cardiotomy filter, i.e., the center inlet tube 150 surrounded by the defoamer tube 154 which in turn is surrounded by the vertical tubular pleated filter 156 secured and sealed at the bottom by the bottom tray 172 and secured and sealed at the top by the potting material 174 is particularly suitable for efficient and reliable manufacture. Additionally, the integral assembly only requires the special manufacture of the filter 30 and cap 48 with the remaining parts being already commercially manufactured for devices being sold without the cardiotomy filter; thus the present integral assembly is particularly economical and efficient for manufacture.

In Fig. 10 there is illustrated a pediatric oxygenator indicated generally at 220 which is essentially the same as the adult oxygenator 20 except that various dimensions of the unit are reduced in size in accordance with the smaller blood flow requirement of a pediatric patient.

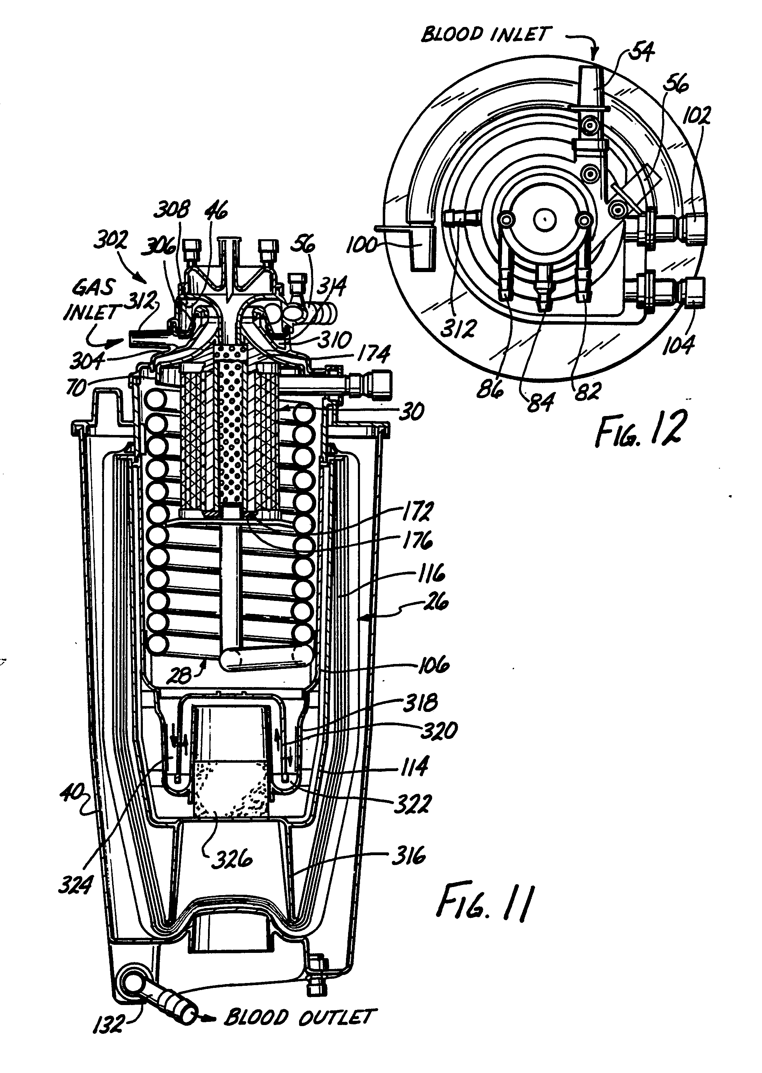

The cardiotomy filter 30 can also be incorporated in bubble oxygenators similar to those disclosed in U.S. Patents 4,282,180 and 4,428,984, as shown by the variation of Figs. 11 and 12. In this variation, bubble oxygenation facilities indicated generally at 302 replace the membrane oxygenation unit 22 of Fig. 1. Various parts are identified in Figs. 11 and 12 by numerals used in Figs. 1-9 to indicate that such parts are similar or perform a similar function. The blood oxygenation facilities are formed by parts 304 and 306 which replace part 44 of Fig. 1. The part 306 divides the chamber between part 304 and 46 into an upper annular chamber 308 and a lower annular chamber 310. The venous blood inlets 54 and 56, formed on part 46, open tangentially into the upper chamber 308 for providing annular blood flow in chamber 308 similar to chamber 64 of Fig. 1. Inlet 312 opens into chamber 310 for supplying pressurized air, oxygen or oxygen-rich gas. Divider part 306 is porous, for example by having holes 314 formed therethrough such as disclosed in U.S. Patent 4,282,180 or 4,297,318, for generating oxygen bubbles in the circulating blood in chamber 308 which is distributed by member 70 to the upper convolutions of coil 28. This blood with gas bubbles cascades down the convolutions of coil 28 which, in the variation of Fig. 10, are shown as being formed from smooth tubing rather than the bellowed tubing shown in Fig. 1. The cardiotomy stream from filter 30 is directed onto the inner surfaces of the coil 28 generally above the vertical midpoint of the coil 28 for being heated as the cardiotomy stream joins the venous stream.

Additionally in the variation shown in Figs. 11 and 12, the reservoir shell 40 is longer, the inner defoamer sock 116 is formed by a plurality of layers of knitted material instead of porous foam, the coil housing 106 has smooth walls rather than being scalloped, and the defoamer support grid 114 has extension 316. Annular trough-like member 318 is secured at its outer lip on the bottom of the coil housing 106, and an inverted cup member 320 has its downward extending annular lip secured on ribs 322 in the bottom of the member 318 for forming an extended passage 324 through which the blood bubble mixture must pass to increase the oxygenation time for the blood. A porous polyurethane foam plug 326 which is coated with a conventional antifoam material is held in the center opening of the annular member 318 by bottom wall of the support grid 114 to receive the flow of blood and bubble mixture. The foam plug 326 reduces the formation of smaller bubbles which could pass through the defoamer 26. After passing through the foam plug 326 to a pool which may form in the reservoir, the bubbles are removed from the blood by contact with the defoamer 26. After passing through the defoamer 26, the blood, now oxygenated, passes through outlet 132.

Many variations, changes in detail and modifications may be made to the described embodiments without departing from the scope and spirit of the invention. It is intended that the above description and the accompanying drawings be interpreted as only illustrative of presently preferred embodiments of the invention, and that the invention as defined in the following claims can also take the form of many other embodiments.

## Claims

1. A reservoir, heating, defoaming and cardiotomy filtering device for an extracorporeal blood system comprising
a bowl-like housing forming a blood reservoir and having a bottom with blood outlet means and a top with venous blood inlet means and with central cardiotomy blood inlet means,
a heat exchanger coil mounted within the housing and having a plurality of vertically arranged convolutions,
said annular venous blood inlet means including means for directing an incoming venous bloodstream in an annular pattern over the upper convolutions of the heat exchanger coil,
a defoamer mounted within the housing and interposed between the heat exchanger and the blood outlet means to remove gas bubbles in the blood passing to the blood outlet,
a cardiotomy filter mounted within the housing and extending downward from the top into the convolutions of the heat exchanger coil,
said cardiotomy filter being connected to said cardiotomy blood inlet means for receiving and filtering a cardiotomy bloodstream, and
means for directing the filtered cardiotomy bloodstream from the cardiotomy filter onto the convolutions of the heat exchanger coil.

2. A device as claimed in claim 1 further comprising means for oxygenating blood.

3. A device as claimed in claim 2 wherein said oxygenating means includes a membrane oxygenator mounted on the bottom of the housing.

4. A device as claimed in claim 1 further comprising a cardiotomy input stream defoamer interposed between the cardiotomy inlet means and the cardiotomy filter.

5. A device as claimed in claim 1 wherein said cardiotomy filter includes a bottom support tray, an annular upper support member, a vertical porous filter tube, means securing the bottom end of the filter tube to the bottom support tray, and means securing the top end of the filter tube to the annular upper support member, said cardiotomy blood inlet means communicating with the interior of the filter tube.

6. A device as claimed in claim 5 wherein said means for directing the filtered cardiotomy bloodstream onto the convolutions of the heat exchanger includes a flexible disc mounted horizontally on the bottom of the bottom support tray and extending into engagement with the heat exchanger coil.

7. A device as claimed in claim 5 further comprising a vertical perforated inlet tube extending from the cardiotomy inlet means to the bottom support tray, and a vertical tubular defoamer pad covering the outer diameter of the inlet tube for defoaming the cardiotomy stream upstream from the filter.

8. A device as claimed in claim 5 including a heating fluid inlet and a heating fluid outlet mounted in the top of the housing, one of said heating fluid inlet and outlet being connected to the topmost convolution of the heat exchanger coil, and a vertical tube section extending vertically along the inside diameter of the heat exchanger coil and connecting the lowermost convolutions of the heat exchanger coil with the other of said heating fluid inlet and outlet, said bottom support tray, said support member and said tubular fibrous filter tube being formed with a vertically extending channel to receive said vertical tube section.

9. A device as claimed in claim 2 wherein said oxygenating means includes means for forming bubbles of oxygen containing gas in the bloodstream within the annular venous blood inlet means.

FIG. 1

EP 0 371 173 A1

FIG. 2

FIG. 3

FIG. 4

FIG. 5

FIG. 6

160
162
164

162
160
164

FIG. 7

148
144
156
150
154
152

FIG. 8

170

FIG. 9

220

FIG. 10

BLOOD INLET 54

56

102

100

312

104

86 84 82

FIG. 12

308 46

302 306 56

GAS INLET 312

304 314

70 310 174

30

172

176

116

26

106

318

320

28

114

322

40

326 316

324

FIG. 11

132 BLOOD OUTLET

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | US-A-4 440 723  (GORDON)<br>* Column 3, lines 8-68; figure 2 *<br>--- | 1 | A 61 M    1/36<br>A 61 M    1/16<br>A 61 M    1/32 |
| A,D | US-A-4 205 042  (LOBDELL et al.)<br>* Column 2, line 53 - column 3, line 4;<br>column 3, lines 31-35; column 4, lines<br>8-23; figure 4 *<br>--- | 1 | |
| A,D | US-A-4 698 207  (BRINGHAM et al.)<br>* Column 4, line 6 - column 5, line 27;<br>figure 1 *<br>--- | 1-3 | |
| A | EP-A-0 122 748  (SHILEY INC.)<br>* Page 5, line 28 - page 6, line 6;<br>page 7, line 23 - page 8, line 27;<br>figure 3 *<br>--- | 1,4,5 | |
| A | US-A-3 701 433  (KRAKAUER et al.)<br>* Column 4, lines 11-45; figures 3,4 *<br>--- | 5 | |
| A | US-A-4 160 801  (BADOLATO et al.)<br>* Column 2, lines 39-66; figure 3 *<br>----- | 1 | TECHNICAL FIELDS SEARCHED (Int. Cl.5)<br><br>A 61 M |

The present search report has been drawn up for all claims

| Place of search | Date of completion of the search | Examiner |
|---|---|---|
| THE HAGUE | 22-06-1989 | SCHOENLEBEN J.E.F. |

EPO FORM 1503 03.82 (P0401)